# EUROPEAN PATENT SPECIFICATION

(11) **EP 2 101 840 B1**
(45) Date of publication and mention of the grant of the patent: **04.09.2013**
(21) Application number: 06829667.2
(22) Date of filing: 15.12.2006
(51) Int. Cl.: A61M 1/10, A61M 1/12

(54) **CARDIOCIRCULATORY AIDING DEVICE**
PUMPE ZUR UNTERSTÜTZUNG DES HERZENS
APPAREIL D'AIDE CARDIOCIRCULATOIRE

(43) Date of publication of application: 23.09.2009
(73) Proprietor: Azzolina, Gaetano, 54016 Licciana Nardi (IT)
(72) Inventor: Azzolina, Gaetano, 54016 Licciana Nardi (IT)
(74) Representative: D'Agostini, Giovanni
(86) International application number: PCT/EP2006/012136
(87) International publication number: WO 2008/071223

(56) References cited:
- EP-A- 1 745 809
- WO-A1-2005/042082
- GB-A- 1 307 135
- GB-A- 2 112 472
- US-A- 3 553 736
- US-A- 4 846 831
- US-A- 4 906 229
- US-A1- 2004 242 954

## Description

### Technical Field

This invention relates to a cardiocirculatory aiding device, namely, to a hematic pump to be associated to the heart whose characteristics are in accordance with the precharacterizing part of the main claim.

### Use domain

The use is substantially directed to assist the heart, but also to replace it in extremis with two of these elements (Right and Left).

### Background art

As known in the cardiosurgery practice, some mechanical aiding devices of the heart are used, commonly defined Ventricular Assist Device (V.A.D.) or Total Artificial Heart (T.A.H.).

We refer substantially to devices able to mechanically pump the blood, producing pulse or continuous hematic flows.

Such devices are used to solve reversible acute cardiac insufficiency clinical cases (e.g.: infarct, myocarditis, morphological pathologies, postcardiotomy, etc...) or are used for supporting the circulatory function awaiting a heart transplantation or even indefinitely in case of situations or irreversible chronic problematic pathologies "Therapy destination".

Different ventricular aid devices have been present for many years, for both left and right aid or biventricular (T.A.H.); some of them are commercially available, other have been developed only to experimental level.

EP 1745809 describes a haematic pump body having an inner space defined by a rigid structure and a pair of mobile membranes alternatively driven in opposite directions by alternatively positive pressure and negative pressure gas supplied in recesses surrounding the two membranes by means of an external pneumatic unit.

GB 1307135 describes an artificial ventricle comprising a rigid outer shell, having pumping chambers formed by membranes bonded to respective inner shells and to the outer shell, the inner shells having apertures to enable pumping fluid to enter the chambers from a pump, situated outside a patient's body. The outer shell has a ventricle cavity with an inlet and outlet comprising male connectors in which are situated one-way flow valves. The pumping chamber comprises a diaphragm secured to the outer shell dividing the pumping chamber from the blood chamber.

US 2004242954 describes a pneumatic ventricular assist device consisting of a soft contoured pumping shell and a disposable pumping unit, which includes a pump sac, two one-way valves, and tubing connectors. The pumping unit is made of a supple and elastic material. The pumping shell consists of shell haves defining the pumping chamber.

GB 2112472 describes a blood pump comprising a housing provided with a fluid port and a cover for closing the housing, a sac defining a blood chamber in the housing, an inlet and outlet blood conduits provided with a check valve and integrally formed on the cover so as to communicate with the blood chamber characterized in that when the blood chamber is compressed, the initial contact point from the opposite inner surfaces of the sac is regulated to locate in the area defined by a circle having the center on the central axis of the wider side of the sac and in the height range 0.2L to 0.45L from the bottom of the sac, the circle radius being 0.15D, where L is the height of the sac, D is the maximum width of the sac and the wider side is defined by the side projected to a vertical plane including D. The compression of the sac is achieved by introduction of air in the housing. WO 2005042082 describes a percutaneous gas-line including a first gas-line and a second gas-line. The first gas-line is wholly implantable in a patient and has a first end adapted for connection to the medical device and a second end with a connection fitting. The second gas-line is part-implanted and part-external and has a first external end for sealing connection to an external driver and a second implanted end for removable sealing connection with the connection fitting on the second end of the first gas-line.

US 4906229 describes a blood pump. The intake and outflow ends of the pump are closed by one-way valves. The pump consists of a stiff barrel whose interior volume can be alternately reduced and expanded by a flexible membrane controlled by pneumatic or hydraulic pressure from an extracorporeal location through a percutaneously inserted lumen. The flexible membrane is sealingly circumferentially attached to the inside wall of the barrel at its intake and outflows ends.

US 4846831 describes a manual back-up emergency device for air driven artificial hearts, to be instantly connected to the air drive lines for the heart in the event of failures and manually actuated during the failure. The device includes a manually actuated pump with a pressure and a suction stroke, and an air delivery tube coupled to a pair of air tubes having quick coupling units on the ends for mating with coupling units on the ends of the air drive tubes to the heart. The device also includes a metering valve in the tube serving the right ventricle of the heart, an instrument reading the metered and pumped air pressures and a make-up air intake in the pump. US3553736 describes an artificial auxiliary ventricle having a generally spherical shape consisting of a rigid casing containing a flexible bulb and directing the flow of blood. The device is driven by compressed air from an outside tank via an air tube and is controlled by a solenoid valve triggered by the ECG or the left ventricular pressure curve. The air pressure is applied between the rigid casing and the flexible bulb.

### Problems and drawbacks of the background art

In the majority of the cases the existing devices have some difficulties of housing inside the chest, due to dimension and weight problems, as well as in the application modalities.

Other noticeable drawbacks are due to their internal geometries and to their pumping modalities that can cause, in respect of the blood, hemolysis or formation of coagulations.

Another negative aspect is caused by the weight and the encumbrance of the operating unit, associated to the pumping device, that limits or precludes the portability of the whole complex.

An additional problem created by the current devices is that they include complex internal mechanisms and, given the complexity, they are not completely reliable.

In fact it is known that the more an apparatus is complex, the more probabilities of jamming danger there are.

Furthermore the well-known devices incorporate electric and/or electromagnetic apparatus, with all the consequences and the dangers thereof.

Finally it is known that such apparatus need more separate groups that must be housed in different positions of the body with further complications and important encumbrances and infection danger.

Furthermore in case of jamming or stopping of the device, there is poor immediate possibility of intervention from outside, endangering the user's life.

### Scope of the invention

The scope of the invention is to solve the above-mentioned problems and drawbacks, by simplifying strongly the apparatus and furthermore:
- to improve the functionality and performances;
- to increase the performance;
- to improve the reliability;
- to adjust easily the apparatus;
- to reduce its encumbrance and its weight;
- to eliminate any electric part to be installed inside of the human body;
- to be able to intervene in case of emergency from the external of the human body without the necessity of complex apparatus, but being able to pump in emergency also with the simple use of the hands;
- use in pediatric cases.

### Solution of the problem and disclosure of the invention

The problem is solved with the characteristics of the main claim.

The subclaims represent advantageous preferred solutions that supply best performance.

### Advantages

In this way, there is the advantage to extremely simplify the apparatus giving the maximum performance guarantee to the user.

Moreover, thanks to the installations of the motor and electric or electronic components, set outside, everything will be easily accessible for maintenance and change and will be also easily adjustable.

A further interesting advantage derives from the fact that the propeller means is a gas (e.g. oxygen or air).

Furthermore, if the pumping external mechanism would clog or stop, a manual pumping means is provided. In fact, a simple small pump connected to the pipe of the gas can be pressed in emergency also by the same user, regularly cadencing the pumping with a sole hand.

The gas loss danger, as propeller means inside of the body is avoided by the current techniques that ensure the gasproof.

In fact the flexible tube of the gas can be carried out in a whole with the respective expanding elastic chamber for example with silicon elastomeric materials (silicon rubber) that ensure a perfect and safe compatibility with the human body, see for example the enormous number of silicon prothesis in the human body (e.g. woman breasts).

For rigid and semirigid structural parts, the dacron can be advantageously used, as already notoriously used in the bypass and in the aortic aneurysms, in valve structures and artificial heartily parts. Naturally also other materials can be used as carbon plastic materials (e.g. pyrocarbon) or also metallic materials e.g. titanium, or any other existing or future technologically suitable and compatible material according to the claims.

### Description of the preferred solution

A preferred embodiment of the cardiocirculatory aiding device according to the invention, will be now described, as a non-limitative example, referring to the drawings in which:

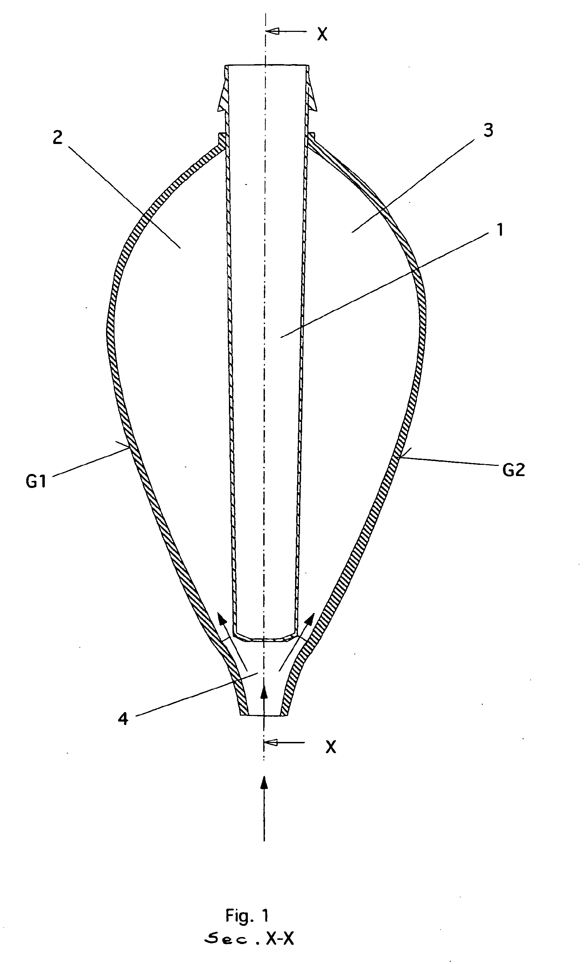

- Figure 1 shows a side sectional view X-X of the device;

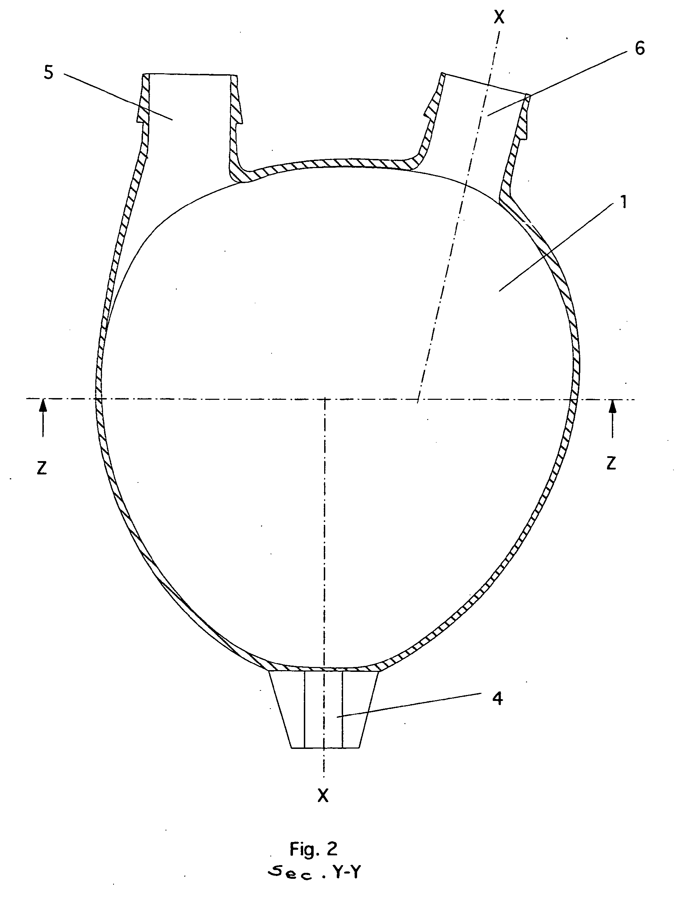

- Figure 2 shows a front sectional view Y-Y of the device;

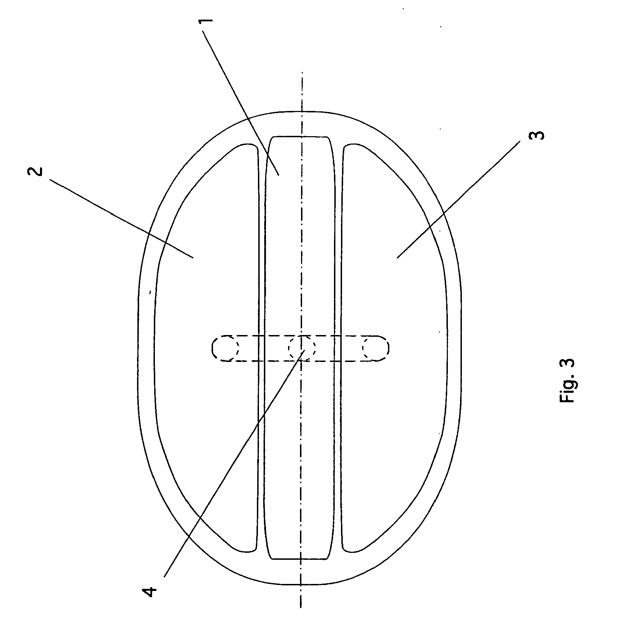

- Figure 3 represents a cross sectional view Z-Z as regards to the previous ones;

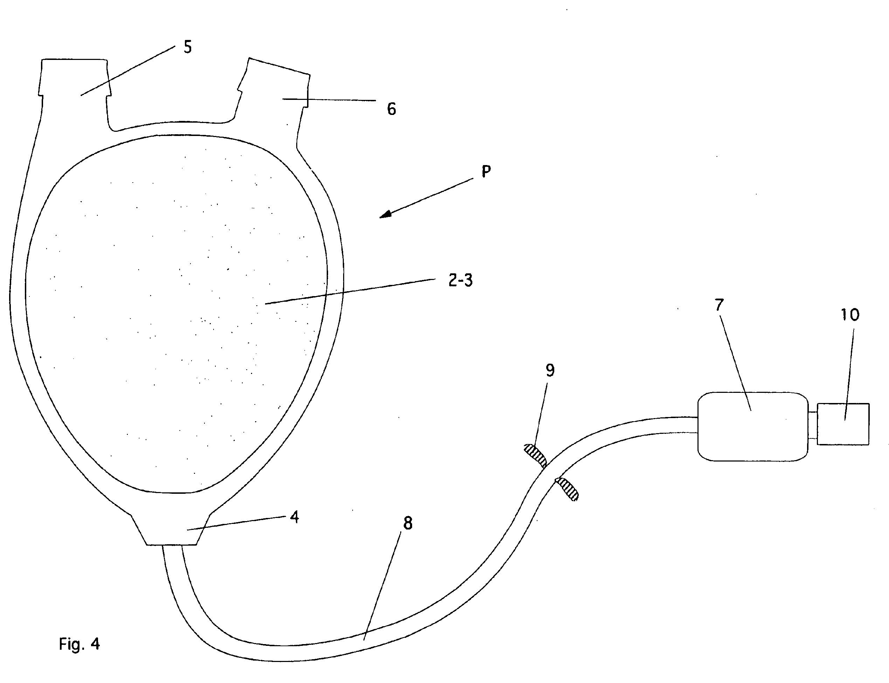

- Figure 4 represents the circuit schema of the device connected to the electro-pneumatic operating unit (7) by means of a tubular duct (8) and the transcutaneous passage (9).

The invention is made up essentially of a core in the form of a semirigid discoidal central chamber **(1)** substantially in DELRIN (hemato-compatible polyammidic resin) with opposite flat walls, which are slightly yielding or flexible, to perform the function of pumping. Inside the central chamber (1) occurs the circulation of blood with the help of two respective in-out ports, that is an opening of blood entry (5) and opening of blood exit (6), with unidirectional valves.

Two elastically flexible opposite chambers (2, 3) for example from silicon elastomer material, that work as two lungs, are used for pressing in a pulsating manner the central chamber (1) giving the action of pulse pumping for deflection of the respective opposite faces, by means of the opening of blood entry (5) and the opening of blood exit (6) associated with the said unidirectional valves, which are technically known (not shown).

Externally, the apparatus appears in the form of a cardioid, wherein in the lower apical zone is a pipe carrier joint (4) for the connection to the pneumatic energy source that must feed the two opposite chambers (2,3). On the opposed side of said pneumatic joint, there are the two blood pumping mouths of opportune diameter: one is used for the entry of the blood, that is the opening of blood entry (5), and the other for the exit, that is the opening of blood exit (6). Both are equipped with respective unidirectional valves not shown.

It is understood in this way that, pressing the central chamber (1), the blood exits from the opening of blood exit (6) being prevented to reflux in the opening of blood entry (5) from the respective one-way valve, whereas the volume of the central chamber (1) returns to its initial position. In this way a classical sucking-pumping pump is obtained, the mechanical parts and gaskets not being subjected to wearing.

The aforementioned mouths, that is the opening of blood entry (5) and the opening of blood exit (6), are manufactured to be easily connected to hemocompatible hoses, commonly used in the cardiochirurgical techniques (e.g. hemocompatible polyammidic resin as DACRON).

A tube connected to the opening of blood entry (5) is used to take the blood from an atrium of the heart (e.g.: by means of atrioventricular cannula) or from other zones of the cardiovascular apparatus; a tube connected to the opening of blood exit (6) is used to restore the blood under pressure to the blood-vessels of the systemic circle or of the pulmonary circle, according to the type of the selected ventricular aid. The internal cavity of the central chamber (1), where the circulation of blood takes place, separates the peripheral zone (inflatable chambers as opposite lungs 2, 3), seat of the pneumatic energy. Said elastically flexible opposite chambers (2, 3), can be of different nature and form, and impress to the blood the necessary pressing energy.

The peripheral zone is closed by means of shells G1 and G2.

The said elastically flexible opposite chambers (2, 3), with pressure increase following gas inlet, inflate and vice-versa, therefore in association with the respective unidirectional valves in the ducts of the blood, the blood pumping by means of the volume variation to the central internal chamber (1) is obtained.

This concerns in conclusion a cardiocirculatory aiding device, in the form of right or left ventricular or biventricular assist device, placeable inside the human body and operated by pulse-gas pneumatic energy, which acts as a closed pump of the type with opposite membrane, but in which there is no membrane (in this specific case, the two opposite walls of the central container 1 act as closed and integrated membranes in the container for safety such as walls of the same container 1), whose pulse is induced by compressed gas (for example oxygen or air) pumped from outside through a thin tubular duct (8) passing through the user's body (9) and transmits the pulses of pressure from respective external motor-driven pump, that is the gas pressure pulses variation generator device (7,10), which is applied externally of the human body and therefore easily controllable and adjustable.

In this way, no electric apparatus is applied inside the organism.

The hematic flow that we can obtain is pulsatile.

The pneumatic energy is therefore characterized by pressing and depressing waves from an external pump, which is necessary for the reciprocating movement of the opposite flexible elastic chambers.

The external pump can be an electro-pneumatic operating unit (7), powered by a battery and/or accumulators (10).

Obviously, the external pump can be of any type, namely piston or membrane operated by cam, etc.

### Further characteristics:

This device has a central chamber (1) which is a semirigid container body, but whose opposite walls can flex for the pumping of the blood, whose internal geometry is shaped hydrodynamically in such a way as to avoid stagnate zones of the blood that may involve the formation of thrombus (internal washing of the ventricular cavity carried out by a rotary flow of the blood).

Therefore, the separating element between the blood and the gas that transmits the pneumatic energy is not only the walls of the central chamber (1), but also the presence of two elastically flexible opposite chambers (2,3), therefore contractible and expandable, performing the pumping function in opposite way to the nucleus having the central sealed chamber (1).

The presence of said elastically flexible opposite chambers (2,3) separating the blood and the gas, whose contraction and constraint modalities impose to said opposite chambers a mechanical stress rationally distributed, results as an advantage for their long-time duration.

The symmetry and the contemporaneity of the movement of the opposite chambers allow to avoid the inertial actions that could make the working of the device flinched.

The electro-pneumatic operating unit (7), availing to known art electro-mechanic devices, results compact and light such as to be transported by the carrying-person of the device, in a small bag, or hooked to a belt.

Such operating unit, that is the gas pressure pulses variation generator device (7-10), rehabilitates the previous realization attempts of pneumatic unit, little used, not for their performances, but for the weight and the encumbrance that up to now have made them prohibitive for portability characteristics.

In fact, the activation by means of pneumatic energy is extremely light and allows to build equally lightweight and of strongly-reduced size aiding devices, unlike the models containing inside electric motors, electromagnetic actuator and other mechanisms.

The aforementioned operating unit can own regulating devices, both manual and automatic, which allow a wide variation of flow emitted by the aiding device.

The beat-frequency imposed to the device can be indicatively between 90 - 180 pulses/min. and can be varied, both manually and automatically, by means of regulating systems present in the electro-pneumatic operating unit 7.

It is understood from the above that the media flow emitted by the device strictly depends on the asynchronous beat-frequency.

Concerning the synchronism deficiency between the natural heart-beats and those of the aiding device, it has been shown, both experimentally and clinically, that the method of the ventricular assistance, by means of highfrequency asynchronous pulses, is not in conflict with the principles of a correct hematic perfusion and how this method does not negatively influence on the behaviour of the assisted natural ventricle.

It has been furthermore verified that, taking into consideration the use of low range devices, the wished media flow values are also reached with the increase of the frequency.

Only following this strategy and depriving the inside of the motor apparatus aiding device, as in the case in object, it is possible to realize elements that for their small size and the low weight, are really implantable inside the chest.

Such affirmations are comforted by the fact that at present aiding devices ventricular based on the principle of the centrifugal pumps exist and have been experimented, which are therefore able to supply only a continuous flow and therefore absolutely out of any possible synchronization with the cardiac pulsation.

While with the method of the continuous artificial perfusion any possibility of synchronism between the diastolic systolic phases of the assisted heart is missing, as regards to those of the aiding device, in case of the asynchronous pulses with high frequency, the physical phenomenon of the beats between the frequency of the assisted heart occurs, as regards to the frequency of the assistance ventricle.

Namely the ventricle of the assisted heart is also periodically in correct relation of phase with the aiding device and, only in such situation, can exhibit a natural reduced pressure range.

Obviously the details can however vary and the components can be built in polymeric material or with substances of other hemocompatible nature.

Said particulars can be joined between them by means of screws, welding, sticking or other methods.

Inside the suction and delivery unidirectional valves, with spontaneous opening, of the type of those used in the valve change surgical installations, it is however possible to use valves of other nature specially manufactured fitted to be housed at the entry of the opening of blood entry (5) and opening of blood exit (6) of the central chamber (1).

More advantageously said unidirectional valves can be housed outside the mouths (5 and 6), that is the opening of blood entry (5) and the opening of blood exit (6), namely said unidirectional valves can be inserted inside the connected hoses to the aforementioned mouths, that is the opening of blood entry (5) and the opening of blood exit (6).

In this way, there is obviously greater operative security and intervention is easier and more silent.

Also advantageously, the core or central chamber (1), whose internal cavity, interested by the hematic flow, can be realized by means of a geometry with reducing section towards the bottom opposite to the ducts (5, 6), that is the opening of blood entry (5) and opening of blood exit (6), that allows the blood to flow without meeting stagnate zones and to be pushed without trauma.

These characteristics allow to avoid, as much as possible, both problems of thrombogenesis and hemolysis.

Also advantageously, the pneumatic connection between the operating unit and the pumping gas engagement (4) occurs by means of the hose or tubular duct (8) that is of small diameter and is built-up in flexible plastic material and endowed with opportune aseptic transcutaneous passage (9).

The electro-pneumatic operating unit (7), produces a pulse gas flushing, whose frequency can be varied both manually and automatically.

Said electro-pneumatic operating unit is supplied by an electric accumulator (10) that can also be housed inside of the unit (7).

Advantageously, the said elastically flexible opposite chambers (2,3) that face on the cavity of the core or central chamber (1), operate substantially as two inversely operating lungs, such to impress to blood a symmetrical push.

From such characteristic, results the fact that the aiding device is without mechanical oscillations or of winces provoked by underbalancing or inertial forces that can be originated from the internal dynamics of the device.

Said elastically flexible opposite chambers (2,3)

can also be interchangeable.

The movable parts are submitted to a mechanical stress uniformly apportioned, which assures a long life.

The pulse device is advantageously asynchronous with the natural heart, to be for delivery for fixed pulse and being able to work to a variable frequency in order to obtain a wide variation of mean flow.

Advantageously, the external pumping device can be completed in the preferred solution, from a very simple emergency manual small pump similar to a flexible pear, with exclusion valve.

In this case, at the jamming of the external pump, it will be sufficient to open the valve of the rubber manual pump and to pump manually.

A circulatory effect on the blood will thus be in that case surely assured in awaiting emergency.

## Claims

1. Cardiocirculatory aiding device, in the form of right or left ventricular or biventricular assist device, placeable inside the human body and operated by pulse-gas pneumatic energy, said device comprising:
i) a gas pressure pulses variation generator device (7,10) intended for being placed outside of the human body;
ii) a blood pumping device (P) configured to be installed inside the human body,
said gas pressure pulses variation generator device (7,10) and pumping device (P) being connected with at least one tubular duct (8) of transmission of said gas, passing from the inside of the human body to the external of the same (9),
said blood pumping device (P) being driven by the variation of pneumatic pressure in said tubular duct (8) by means of pressure means (2,3) effective on a central chamber (1) of said pumping device by pulses, in such a way as to circulate the blood, by means of two openings one of blood entry (5) and one of blood exit (6) each associated with a unidirectional valve,
said pressure means (2, 3) consisting in two expansible and contractible elastically flexible opposite chambers (2,3) such as to transmit the pulse of the gas to said central chamber (1) of the blood pumping device **characterized in that**
said central chamber (1) is a semirigid distinct container body configured and structured as a core in the form of a semirigid discoidal central chamber with opposite flat walls, which are slightly yielding or flexible and has only one entry duct (5) and only one exit duct (6), said central chamber (1) separating a peripheral zone consisting of said two elastically flexible opposite chambers (2,3) having a symmetric and contemporary movement which transmits the pulse gas pneumatic energy to said central chamber (1) of the blood pumping device, said opposite flat walls being slightly pressed and flexed by the inflation of said two opposite chambers (2,3), the separating element between the blood and the gas that transmits the pneumatic energy being the walls of said central chamber (1)
and further **characterized in that** it comprises a flexible small manual pump, with an exclusion valve and with connection to the said duct (8) for operating as an alternative to said gas pressure pulses variation generator device (7,10) for pumping of the gas, the opening of said exclusion valve being intended to put into communication said flexible small manual pump with said duct (8) to manually pump the gas with a sole hand.

2. Cardiocirculatory aiding device, according to claim 1, **characterized in that** said unidirectional valves are external to the said pump (P), namely applied in the respective entry and exit ducts of the blood applied to said respective opening of blood entry (5) and opening of blood exit (6).

3. Cardiocirculatory aiding device, according to claim 1, **characterized in that** said unidirectional valves are incorporated in said respective opening of blood entry (5) and opening of blood exit (6) of the pumping device (P).

4. Cardiocirculatory aiding device, according to any of claims 1 to 3, **characterized in that** said central chamber (1) has substantially a discoidal section, progressively decreasing at the opposite side of said opening of blood entry (5) and opening of blood exit (6).

5. Cardiocirculatory aiding device, according to any of claims 1 to 4, **characterized in that** said tubular duct (8) is made of flexible plastic material.

6. Cardiocirculatory aiding device, according to any of claims 1 to 5, **characterized in that** said gas pressure pulse variation generator device (7,10) is an electro-pneumatic operating unit (7), that produces a pulse gas flushing whose frequency can be varied both manually and automatically.

7. Cardiocirculatory aiding device, according to any of claims 1 to 6, **characterized in that** the said elastically flexible opposite chambers (2,3) consist of elastic chambers that face on the cavity of the central chamber (1), such to impress to the blood a symmetrical push.

8. Cardiocirculatory aiding device, according to claim 7, **characterized in that** said elastically flexible opposite chambers (2,3) are movable, flexible and interchangeable.

9. Cardiocirculatory aiding device, according to any of claims 1 to 8, **characterized in that** said gas pressure pulses variation generator device (7,10) is asynchronous with the natural heart and is adjustable to variable frequency.

## Patentansprüche

1. Herz-Kreislauf-Hilfsvorrichtung in Form eines rechten oder linken ventrikulären oder biventrikulären Kunstherzens, das im menschlichen Körper platzierbar ist und durch pneumatische Impulsgasenergie betrieben wird, wobei besagte Vorrichtung folgendes umfasst:
i) einen Gasdruck-Pulsvariationsgenerator (7,10), der außerhalb des menschlichen Körpers platziert wird;
ii) eine Blutpumpe (P), die im menschlichen Körper installiert wird,
wobei der Gasdruck-Pulsvariationsgenerator (7,10) und die Pumpvorrichtung (P) mit mindestens einer Rohrleitung (8) zur Übertragung des Gases verbunden sind, die vom Innern des menschlichen Körpers nach außen (9) führt,
wobei die Blutpumpe (P) durch die Veränderung des pneumatischen Drucks in besagter Rohrleitung (8) mittels Druckmittel (2,3), die auf eine zentrale Kammer (1) der Pumpvorrichtung einwirken, durch Impulse angetrieben wird, damit das Blut zirkuliert, mittels zweier Öffnungen, eine zum Bluteintritt (5) und eine zum Blutaustritt (6), die jeweils mit einem Rückschlagventil verbunden sind,
besagte Druckmittel (2,3) bestehen aus zwei dehnbaren und zusammenziehbaren elastisch flexiblen gegenüberliegenden Kammern (2,3), um den Impuls des Gases auf die zentrale Kammer (1) der Blutpumpe zu übertragen,
**gekennzeichnet dadurch, dass**
die zentrale Kammer (1) ein halbstarrer separater Behälter ist, der als ein Kern in Form einer halbstarren scheibenförmigen zentralen Kammer mit gegenüberliegenden flachen Wänden konfiguriert und strukturiert ist, die leicht nachgebend oder flexibel sind und nur eine Eintrittsleitung (5) und nur eine Austrittsleitung (6) haben, wobei die zentrale Kammer (1) einen Randbereich abtrennt, bestehend aus den beiden elastisch flexiblen gegenüberliegenden Kammern (2,3) mit einer symmetrischen und gleichzeitigen Bewegung, die die pneumatische Impulsgasenergie zu der zentralen Kammer (1) der Blutpumpe überträgt, wobei die gegenüberliegenden flachen Wände durch das Aufblasen der beiden gegenüberliegenden Kammern (2,3) leicht gedrückt und gebogen werden, wobei das trennende Element zwischen dem Blut und dem Gas, das die pneumatische Energie überträgt, die Wände der zentralen Kammer (1) sind,
und weiter **gekennzeichnet dadurch, dass** sie eine flexible kleine manuelle Pumpe umfasst, mit einem Ausschlussventil und mit einer Verbindung zu der Leitung (8), um als Alternative zu dem Gasdruck-Pulsvariationsgenerator (7,10) zum Pumpen des Gases zu dienen, wobei die Öffnung des Ausschlussventils dazu dient, die flexible kleine manuelle Pumpe mit der Leitung (8) in Verbindung zu setzen, um das Gas mit nur einer Hand zu pumpen.

2. Herz-Kreislauf-Hilfsvorrichtung gemäß Anspruch 1, **gekennzeichnet dadurch, dass** die Rückschlagventile außerhalb der besagten Pumpe (P) liegen, und zwar in den betreffenden Blutein- und -austrittsleitungen, die an der Bluteintrittsöffnung (5) und Blutaustrittsöffnung (6) angebracht sind.

3. Herz-Kreislauf-Hilfsvorrichtung gemäß Anspruch 1, **gekennzeichnet dadurch, dass** die Rückschlagventile in der Bluteintrittsöffnung (5) und Blutaustrittsöffnung (6) der Pumpvorrichtung (P) eingebaut sind.

4. Herz-Kreislauf-Hilfsvorrichtung nach einem beliebigen der Ansprüche 1 bis 3, **gekennzeichnet dadurch, dass** besagte zentrale Kammer (1) im wesentlichen einen scheibenförmigen- Querschnitt hat, der sich an der Gegenseite der Bluteintrittsöffnung (5) und Blutaustrittsöffnung (6) fortschreitend verringert.

5. Herz-Kreislauf-Hilfsvorrichtung nach einem beliebigen der Ansprüche 1 bis 4, **gekennzeichnet dadurch, dass** besagte Leitung (8) aus flexiblem Kunststoff besteht.

6. Herz-Kreislauf-Hilfsvorrichtung nach einem beliebigen der Ansprüche 1 bis 5, **gekennzeichnet dadurch, dass** der Gasdruckimpulsveränderungs-Generator (7,10) eine elektropneumatische Betriebseinheit (7) ist, die eine Impulsgasspülung erzeugt, deren Frequenz sowohl manuell als auch automatisch variiert werden kann.

7. Herz-Kreislauf-Hilfsvorrichtung nach einem beliebigen der Ansprüche 1 bis 6, **gekennzeichnet dadurch, dass** die elastisch flexiblen einander gegenüberliegenden Kammern (2,3) aus elastischen Kammern bestehen, die dem Hohlraum der zentralen Kammer (1) gegenüberliegen, um dem Blut einen symmetrischen Schub zu verleihen.

8. Herz-Kreislauf-Hilfsvorrichtung gemäß Anspruch 7, **gekennzeichnet dadurch, dass** die elastisch flexiblen einander gegenüberliegenden Kammern (2,3) beweglich, flexibel und auswechselbar sind.

9. Herz-Kreislauf-Hilfsvorrichtung, nach einem beliebigen der Ansprüche 1 bis 8, **gekennzeichnet dadurch, dass** der Gasdruck-Pulsvariationsgenerator (7,10) asynchron mit dem natürlichen Herzen und frequenzregelbar ist.

## Revendications

1. Dispositif d'aide cardio-circulatoire sous la forme d'un dispositif d'assistance biventriculaire ou ventriculaire gauche ou droit, plaçable dans le corps humain et fonctionnant par énergie pneumatique au gaz à impulsion, ledit dispositif comprenant :
i) un générateur de variation de pulsations de pression de gaz (7, 10) destiné à être placé en dehors du corps humain ;
ii) un dispositif de pompage de sang (P) configuré pour être installé dans le corps humain,
ledit dispositif générateur de variation de pulsations de pression de gaz (7, 10) et le dispositif de pompage (P) étant reliés avec au moins un conduit tubulaire (8) de transmission dudit gaz, qui passe de l'intérieur du corps humain à l'extérieur de celui-ci (9),
ledit dispositif de pompage de sang (P) étant actionné par la variation de la pression pneumatique dans ledit conduit tubulaire (8) par l'intermédiaire de moyens de pression (2, 3) qui agissent sur une chambre centrale (1) dudit dispositif de pompage par impulsions, de façon à faire circuler le sang, au moyen de deux ouvertures, l'une d'entrée du sang (5) et l'autre de sortie du sang (6) toute deux associées à une valve unidirectionnelle,
lesdits moyens de pression (2, 3) consistant en deux chambres opposées expansibles et contractiles, et élastiquement flexibles (2, 3) de façon à transmettre l'impulsion du gaz à ladite chambre centrale (1) du dispositif de pompage de sang
**caractérisé en ce que**
ladite chambre centrale (1) est un récipient distinct semi-rigide, structuré et configuré comme un noyau sous la forme d'une chambre centrale discoïdale semi-rigide avec des parois plates opposées, qui sont légèrement souples ou flexibles et qui ont seulement un conduit d'entrée (5) et seulement un conduit de sortie (6), ladite chambre centrale (1) séparant une zone périphérique, composée desdites deux chambres opposées élastiquement flexibles (2, 3) ayant un mouvement synchrone et symétrique qui transmet l'énergie pneumatique de gaz à impulsion à ladite chambre centrale (1) du dispositif de pompage de sang, lesdites parois plates opposées étant légèrement pressées et pliées par l'inflation desdites deux chambres opposées (2, 3), l'élément de séparation entre le sang et le gaz qui transmet l'énergie pneumatique étant les parois de ladite chambre centrale (1)
et en outre **caractérisé en ce qu'**il comprend une petite pompe manuelle flexible, avec une valve d'exclusion et avec une connexion audit conduit (8) pour fonctionner en alternative audit dispositif générateur de variation de pulsations de pression de gaz (7, 10) pour pomper le gaz, l'ouverture de ladite valve d'exclusion étant destinée à mettre en communication ladite petite pompe manuelle flexible avec ledit conduit (8) pour pomper manuellement le gaz avec une seule main.

2. Dispositif d'aide cardio-circulatoire selon la revendication 1, **caractérisé en ce que** lesdites valves unidirectionnelles sont externes à la ladite pompe (P), c'est-à-dire appliquées dans les conduits d'entrée et de sortie du sang appliqués à ladite ouverture d'entrée du sang (5) et à l'ouverture de sortie du sang (6).

3. Dispositif d'aide cardio-circulatoire, selon la revendication 1, **caractérisé en ce que** lesdites valves unidirectionnelles sont incorporées dans ladite ouverture d'entrée du sang (5) et dans l'ouverture de sortie du sang (6) du dispositif de pompage (P).

4. Dispositif d'aide cardio-circulatoire, selon l'une quelconque des revendications 1 à 3, **caractérisé en ce que** ladite chambre centrale (1) a substantiellement une section discoïdale, progressivement décroissante du côté opposé desdites ouverture d'entrée du sang (5) et ouverture de sortie du sang (6).

5. Dispositif d'aide cardio-circulatoire, selon l'une quelconque des revendications 1 à 4, **caractérisé en ce que** ledit conduit tubulaire (8) est en matière plastique flexible.

6. Dispositif d'aide cardio-circulatoire, selon l'une quelconque des revendications 1 à 5, **caractérisé en ce que** ledit dispositif générateur de variation d'impulsion de pression de gaz (7, 10) est une unité à fonctionnement électropneumatique (7) qui produit un flux de gaz à impulsion dont la fréquence peut être variée soit manuellement soit automatiquement.

7. Dispositif d'aide cardio-circulatoire, selon l'une quelconque des revendications 1 à 6, **caractérisé en ce que** lesdites chambres opposées élastiquement flexibles (2, 3) consistent en des chambres élastiques dirigées sur la cavité de la chambre centrale (1) afin de conférer au sang une propulsion symétrique.

8. Dispositif d'aide cardio-circulatoire, selon la revendication 7, **caractérisé en ce que** lesdites chambres opposées élastiquement flexibles (2, 3) sont mobiles, interchangeables et flexibles.

9. Dispositif d'aide cardio-circulatoire, selon l'une quelconque des revendications 1 à 8, **caractérisé en ce que** ledit générateur de variation de pulsations de pression de gaz (7, 10) est asynchrone avec le coeur naturel et est réglable à fréquence variable.
